# FASCICULE DE BREVET EUROPEEN

(11) **EP 2 334 245 B1**
(45) Date de publication et mention de la délivrance du brevet: **07.12.2016**
(21) Numéro de dépôt: 09756159.1
(22) Date de dépôt: 02.10.2009
(51) Int. Cl.: A61B 17/80

(54) **IMPLANT ORTHOPEDIQUE SOUS FORME D'UNE PLAQUE DESTINEE A ETRE FIXEE ENTRE DEUX PARTIES D'OS**
ORTHOPÄDISCHES IMPLANTAT IN FORM EINER PLATTE ZUR FIXIERUNG ZWISCHEN ZWEI KNOCHENTEILEN
ORTHOPEDIC IMPLANT IN THE FORM OF A PLATE TO BE FIXED BETWEEN TWO BONE PARTS

(30) Priorité: 02.10.2008 FR 0856694
(43) Date de publication de la demande: 22.06.2011
(73) Titulaire: Stryker European Holdings I, LLC, Kalamazoo, MI 49002 (US)
(72) Inventeur: PRANDI, Bernard, F-35700 Rennes (FR); WAPNER, Keith, Philadelphie, PA 19103 (US); WAPNER, Peter W, Pennsylvania 19063 (US); WAPNER Charles P, Pennsylvania 19063 (US)
(74) Mandataire: Regimbeau
(86) Numéro de dépôt international: PCT/FR2009/051879
(87) Numéro de publication internationale: WO 2010/037985

(56) Documents cités:
- WO-A1-02/098306
- DE-A1- 3 630 862
- DE-U1- 8 227 727
- FR-A- 590 290
- US-A1- 2005 070 904
- US-A1- 2008 114 360

## Description

L'invention se rattache au secteur technique des implants orthopédiques.

Plus particulièrement, l'invention concerne une plaque pour arthrodèse ou ostéosynthèse destinée à être fixée entre deux parties d'os.

D'une manière parfaitement connue pour un homme du métier, ce type de plaque comprend, généralement, des trous pour l'engagement de vis permettant de réaliser une arthrodèse entre deux os ou une ostéosynthèse entre deux fragments osseux. C'est le cas, par exemple, pour les os de la main ou du pied, sans pour cela exclure d'autres applications, notamment dans le domaine du rachis. En fonction du cas pathologique à traiter, ces plaques peuvent être de forme générale rectiligne ou présenter d'autres formes géométriques.

Le document WO02098306 A1 décrit un implant orthopédique ayant une structure sous forme d'une plaque définissant un plan destiné à être fixée entre une première et une seconde partie d'os au moyen de vis engagées dans des premier et deuxième trous de fixation.

A partir de cet état de la technique, l'un des problèmes que se propose de résoudre l'invention est d'améliorer, d'une manière sure et efficace, la compression entre les parties d'os assujetties à la plaque et selon une direction précise.

Pour résoudre le problème posé d'améliorer la compression entre les deux parties d'os considérées, selon l'invention, la plaque présente au moins un agencement apte à permettre de positionner au moins une vis d'une manière inclinée par rapport au plan défini par ladite plaque selon un angle compris entre 30° et 60° environ.

Selon une forme avantageuse de réalisation, l'agencement est constitué par une zone inclinée selon l'angle compris entre 30° et 60°, et présentant un trou pour l'engagement de la vis. La zone inclinée résulte d'une découpe et d'une déformation d'une partie de la plaque.

Dans une autre forme de réalisation, l'agencement est constitué par un trou incliné selon l'angle compris entre 30° et 60° pour l'engagement de la vis.

Compte tenu du problème posé à résoudre, l'agencement est situé sur une partie déterminée de la longueur de la plaque pour que la vis assure la mise en compression des deux parties d'os.

L'invention est exposée ci-après plus en détail à l'aide des figures des dessins annexés dans lesquels :
- la figure 1 est une vue en perspective d'une forme de réalisation de la plaque;
- la figure 2 est une vue de profil de la plaque;
- les figures 3 et 4 sont des vues en perspective montrant le montage de la plaque entre deux parties d'os et la composition de ces dernières, au moyen de la plaque selon l'invention, les parties d'os étant présentées d'une manière schématique.

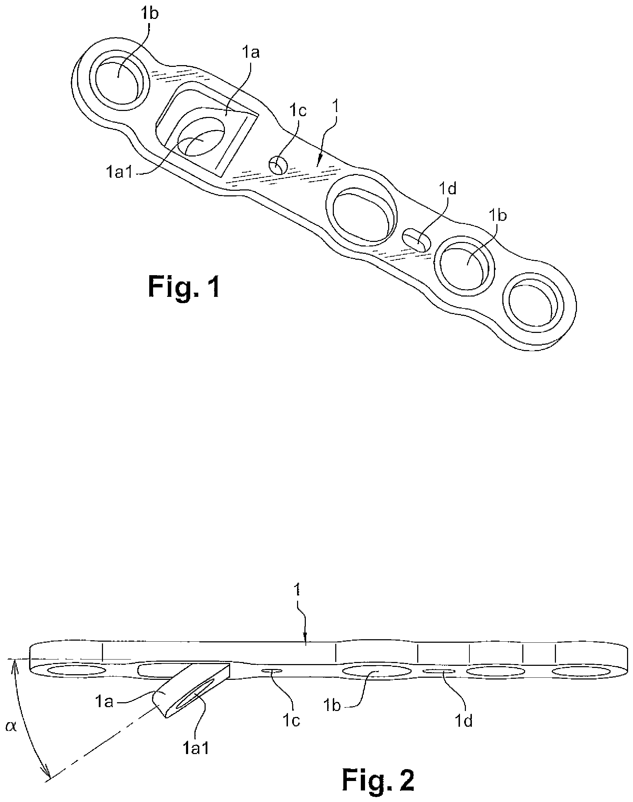

Selon l'invention, la plaque (1) présente au moins un agencement (1a) apte à permettre de positionner au moins une vis (2), d'une manière inclinée, selon un angle α compris entre 30° et 60° par rapport au plan défini par ladite plaque (figure 2)

Dans une forme de réalisation, l'agencement (1a) est constitué par une zone inclinée qui résulte d'une découpe et d'une déformation d'une partie de la plaque. Par exemple, la déformation est consécutive à une opération de découpage - poinçonnage. Cette zone inclinée constitue une nervure qui présente un trou (1a1) pour l'engagement de la vis (2). La nervure inclinée (1a) est formée sur une partie déterminée de la longueur de la plaque pour qu'après engagement, la vis (2) assure la mise en compression des deux parties d'os, comme il sera indiqué dans la suite de la description.

Dans une autre forme de réalisation, pour permettre une orientation angulaire de la vis (2), selon un angle compris entre 30° et 60° environ, l'agencement (1a) peut être constitué par un trou incliné. A noter que la nervure (1a) permet une adaptation de l'angle en fonction du cas pathologique à traiter étant donné qu'il est possible de déformer à volonté cette nervure. Autrement dit, l'angle peut être réglé directement par le chirurgien sur quelques degrés en bloc opératoire avec un instrument adapté.

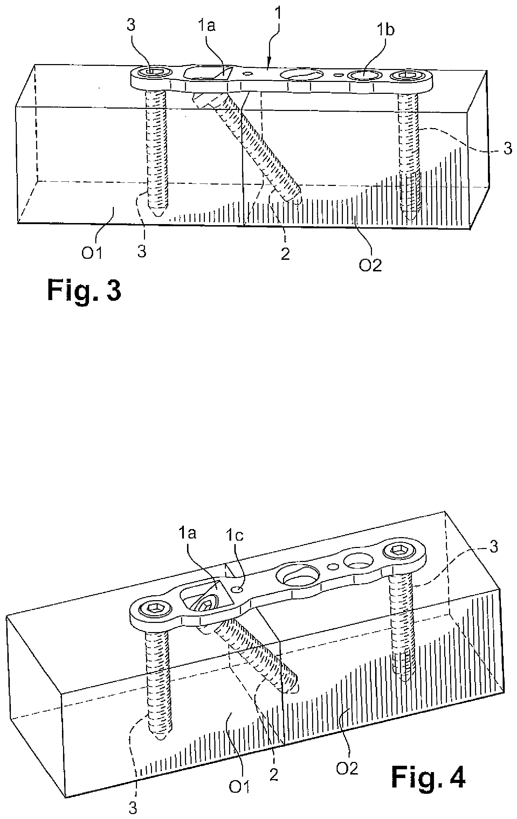

On renvoie aux figures 3 et 4 qui montrent le positionnement de la plaque (1) entre deux parties d'os (O1) et (O2):
- Après réalisation des ostéotomies, un gabarit de la plaque, qui ne présente pas de nervures, permet de déterminer la position de cette nervure.
- Après avoir déterminé le positionnement de la nervure, le chirurgien réalise un logement correspondant, avec une râpe adaptée.
- Après positionnement de la plaque présentant la nervure, le chirurgien met en place une ou deux vis (3), d'un côté du foyer d'ostéosynthèse de l'arthrodèse considéré du côté de la nervure. On peut positionner, éventuellement, une broche de fixation temporaire dans un plot adapté.
- La vis (2) est ensuite engagée dans le trou (1a1) de la nervure (1a) pour mettre le foyer de fracture en compression.
- Une fois la compression effectuée, le chirurgien peut visser une ou plusieurs autres vis (3) de fixation complémentaire et retirer la broche temporaire de maintien

D'une manière connue, cette plaque (1) présente des trous lisses et/ou taraudés (1b) pour l'engagement des vis de fixation (3) vissées dans les parties d'os (O1) et (02), comme il ressort des figures 3 et 4.

De même, la plaque (1) peut présenter au moins un logement (1c) pour l'introduction d'une broche en vue d'assurer une fixation temporaire de ladite plaque (1). Avantageusement, la plaque (1) peut présenter un logement (1c) pour l'introduction d'une broche du côté de l'une des parties de l'os (O1) et un autre logement (1d) pour l'introduction d'une autre broche du côté de l'autre partie d'os (02).

Compte tenu de l'effet de compression recherchée, telle qu'indiquée précédemment, le logement (1c) est constitué par un trou circulaire dont le diamètre correspond sensiblement à celui de la broche (4), tandis que l'autre logement (1d) peut être constitué par une lumière oblongue.

Ces dispositions permettent donc à l'os de coulisser sous la plaque (1) au moment du vissage, tout en assurant une compression selon une direction précise, généralement suivant l'axe de la plaque. Les broches sont de tout type connu et approprié, et parfaitement connu pour un homme du métier.

La plaque (1) peut présenter différentes formes géométriques, de sorte que les trous (1a) notamment peuvent être alignés ou être disposés, en totalité ou en partie, selon les sommets d'un triangle ou d'un quadrilatère. Ces dispositions, en triangle ou en quadrilatère des vis, améliorent la stabilité du montage.

A noter également que la plaque (1), quelle que soit sa forme géométrique, peut être cintrée longitudinalement, pour s'adapter à la courbure de l'os permettant, en conséquence, aux vis (2) de former un angle entre elles.

Les avantages ressortent bien de la description.

## Revendications

1. Implant orthopédique ayant une structure sous forme d'une plaque (1) définissant un plan destiné à être fixée entre une première et une seconde partie d'os au moyen de vis engagées dans des premier et deuxième trous (1b) de fixation, lesdits premier et deuxième trous de fixation étant situés à deux emplacements séparés de la plaque, ladite plaque comprenant de plus un troisième trou (1a1) situé entre lesdits premier et deuxième trous de fixation (1b) pour l'engagement d'une vis de compression (2), lesdits premier et deuxième trous de fixation (1b) étant formés dans l'épaisseur de la plaque, la plaque présentant un agencement formé par une nervure (1a) présentant le troisième trou (1a1), ledit troisième trou étant incliné de façon à permettre de positionner la vis de compression (2) au travers dudit troisième trou (1a1) d'une manière inclinée par rapport au plan de ladite plaque selon un angle compris entre 30° et 60°, l'agencement étant situé dans une partie médiane, en largeur, sur une partie déterminée de la longueur de la plaque pour que la vis de compression (2) assure la mise en compression entre la première et la seconde partie d'os, dans lequel la nervure (1a) fait saillie par rapport au plan de ladite plaque du (1) et ledit troisième trou (1a1) est par conséquent positionné en dehors du plan de ladite plaque (1), le premier trou de fixation (1b) étant agencé pour diriger une première vis dans la première partie d'os, et le deuxième trou de fixation (1b) étant agencé pour diriger une seconde vis dans ladite seconde partie d'os.

2. Implant selon la revendication 1, **caractérisé en ce que** la plaque présente un logement (1c) agencé pour l'introduction d'une broche de fixation dans la première partie d'os, et un autre logement (ld) agencé pour l'introduction d'une broche dans la seconde partie d'os.

3. Implant selon la revendication 2, **caractérisé en ce que** l'un des logements est constitué par un trou circulaire (1c) dont le diamètre correspond sensiblement à celui de la broche, tandis que l'autre logement est constitué par une lumière oblongue (1d).

4. Implant selon l'une des revendications 1 à 3, **caractérisé en ce que** la plaque présente des trous lisses et/ou taraudés destinés à recevoir des vis de fixation avec les parties d'os.

5. Implant selon la revendication 4, **caractérisé en ce que** les différents trous sont alignés.

6. Implant selon la revendication 4, **caractérisé en ce que** certains des trous sont disposés selon le sommet d'un triangle ou d'un quadrilatère.

7. Implant selon l'une quelconque des revendications précédentes, **caractérisé en ce que** la plaque (1) est cintrée longitudinalement pour s'adapter à la courbure de l'os, permettant en conséquence aux vis de former un angle entre elles.

8. Implant selon l'une quelconque des revendications précédentes, **caractérisé en ce que** la plaque (1) est agencée pour permettre la jonction par compression entre les parties d'os par insertion d'une vis de compression (2) au travers du troisième trou (1a1) situé dans la partie médiane.

9. Implant selon l'une quelconque des revendications précédentes, **caractérisé en ce que** la nervure (1a) et le troisième trou (1a1) situé dans la partie médiane sont situés en dessous d'un trou de fixation parmi les premier et deuxième trous de fixation (1b) de la plaque.

10. Implant selon l'une quelconque des revendications précédentes, **caractérisé en ce que** les premier et deuxième trous de fixation (1b) de la plaque sont taraudés.

11. Implant selon l'une quelconque des revendications précédentes, **caractérisé en ce que** des trous de fixation additionnels sont prévus dans l'épaisseur de la plaque (1) entre ou à l'extérieur des premier et deuxième trous de fixation (1b).

## Patentansprüche

1. Orthopädisches Implantat, das eine Struktur in Form einer Platte (1), die eine Ebene zur Befestigung zwischen einem ersten und einem zweiten Teil des Knochens mittels von von der ersten und zweiten Befestigungsöffnung (1b) aufgenommenen Schrauben ausbildet, wobei die ersten und zweiten Befestigungsöffnungen an zwei gesonderten Stellen der Platte angeordnet sind, die Platten zudem eine zwischen der ersten und zweiten Befestigungsöffnung (1b) angeordnete dritte Öffnung (1a1) für die Aufnahme einer Kompressionsschraube (2) aufweist, wobei die erste und zweite Befestigungsöffnung (1b) in der Dicke der Platte ausgebildet ist und die Platte einen durch eine die dritte Öffnung (1a1) aufweisende Rippe (1a) ausbildenden Aufbau darstellt, die dritte Öffnung derart geneigt ist, dass die Anordnung der Kompressionsschraube (2) über die dritte Öffnung (1a1) in geneigter Form in Bezug auf die Ebene dieser Platte nach einem Winkel zwischen 30° und 60° ermöglicht wird, wobei der Aufbau in einem breitenmäßigen Mittelteil auf einem bestimmten Teil der Länge der Platte angeordnet ist, damit die Kompressionsschraube (2) die Kompression zwischen dem ersten und dem zweiten Knochenteil gewährleistet, bei der die Rippe (1a) in Bezug auf die Ebene dieser Platte (1) hervorsteht und die dritte Öffnung (1a1) dementsprechend außerhalb der Ebene dieser Platte (1) angeordnet ist, wobei die erste Befestigungsöffnung (1b) ausgeführt wird, um eine erste Schraube in einem ersten Knochenteil zu lenken, und die zweite Befestigungsöffnung (1b) ausgeführt ist, um eine zweite Schraube in diesem zweiten Knochenteil zu lenken.

2. Implantat nach Anspruch 1, **dadurch gekennzeichnet, dass** die Platte eine Öffnung (1c), die für die Aufnahme einer Befestigungsspindel im ersten Knochenteil aufgebaut ist, und eine zweite Öffnung (1d), die für die Einführung einer Spindel in den zweiten Knochenteil aufgebaut ist, aufweist.

3. Implantat nach Anspruch 2, **dadurch gekennzeichnet, dass** eine der Öffnungen aus einer kreisförmigen Öffnung (1c) ausgebildet ist, deren Durchmesser im Wesentlichen dem der Spindel entspricht, während die andere Öffnung aus einem länglichen Licht (1d) gebildet wird.

4. Implantat nach Anspruch 1 bis 3, **dadurch gekennzeichnet, dass** die Platte glatte und/oder Gewindeöffnungen zur Aufnahme der Befestigungsschrauben mit den Knochenteilen aufweist.

5. Implantat nach Anspruch 4, **dadurch gekennzeichnet, dass** die unterschiedlichen Öffnungen aneinandergereiht sind.

6. Implantat nach Anspruch 4, **dadurch gekennzeichnet, dass** die Öffnungen nach der Spitze eines Drei- oder Vierecks angeordnet sind.

7. Implantat nach einem der vorstehenden Ansprüche, **dadurch gekennzeichnet, dass** die Platte (1) längs gebogen ist, um sich an die Rundung des Knochens anzupassen, was den Schrauben demzufolge ermöglicht, untereinander einen Winkel auszubilden.

8. Implantat nach einem der vorstehenden Ansprüche, **dadurch gekennzeichnet, dass** die Platte (1) aufgebaut wird, um die Verbindung durch Kompression zwischen den Knochenteilen durch die Einfügung einer Kompressionsschraube (2) über die im Mittelteil angeordnete dritte Öffnung (1a1) zu ermöglichen.

9. Implantat nach einem der vorstehenden Ansprüche, **dadurch gekennzeichnet, dass** die Rippe (1a) und die im Mittelteil angeordnete dritte Öffnung (1a1) über einer Befestigungsöffnung unter der ersten und zweiten Befestigungsöffnung (1b) der Platte ausgeführt sind.

10. Implantat nach einem der vorstehenden Ansprüche, **dadurch gekennzeichnet, dass** die erste und zweite Befestigungsöffnung (1b) der Platte gewindegeschnitten ist.

11. Implantat nach einem der vorstehenden Ansprüche, **dadurch gekennzeichnet, dass** zusätzliche Befestigungsöffnungen in der Dicke der Platte (1) zwischen oder außerhalb der ersten und zweiten Befestigungsöffnung (1b) vorgesehen sind.

## Claims

1. An orthopedic implant having a structure in the form of a plate (1) defining a plane intended to be fixed between a first and a second bone part by means of screws engaged in first and second attachment holes (1b), said first and second attachment holes being located at two separate locations of the plate, said plate additionally comprising a third hole (1a1) located between said first and second attachment holes (1b) for engaging a compression screw (2), said first and second attachment holes (1b) being formed in the thickness of the plate, the plate having an arrangement formed by a rib (1a) having the third hole (1a1), said third hole being inclined so as to allow positioning the compression screw (2) through said third hole (1a1) in a manner that is inclined with respect to the plane of said plate at an angle comprised between 30° and 60°, the arrangement being located in a middle part, in width, on a predetermined part of the length of the plate so that the compression screw (2) provides for compression between the first and the second bone part, wherein the rib (1a) protrudes with respect to the plane of said plate (1) and said third hole (1a1) is consequently positioned outside the plane of said plate (1), the first attachment hole (1b) being arranged to direct a first screw into said first bone part, and the second attachment hole (1b) being arranged to direct a second screw into said second bone part.

2. The implant according to claim 1, **characterized in that** the plate has a hole (1c) arranged for the introduction of an attachment pin into the first bone part, and another housing (1d) arranged for the introduction of a pin into the second bone part.

3. The implant according to claim 2, **characterized in that** one of the holes consists of a circular hole (1c), the diameter whereof corresponds substantially to that of the pin, while the other housing consists of an oblong opening (1d).

4. The implant according to one of claims 1 to 3, **characterized in that** the plate has smooth and/or tapped holes intended to receive screws for attachment to the bone parts.

5. The implant according to claim 4, **characterized in that** the different holes are aligned.

6. The implant according to claim 4, **characterized in that** some of the holes are arranged along the apex of a triangle or a quadrilateral.

7. The implant according to any one of the preceding claims, **characterized in that** the plate (1) is curved longitudinally so as to adapt to the curvature of the bone, consequently allowing the screws to form an angle between them.

8. The implant according to any one of the preceding claims, **characterized in that** the plate (1) is arranged to allow connection by compression between the bone parts by the insertion of a compression screw (2) through the third hole (1a1) located in the middle part.

9. The implant according to any one of the preceding claims, **characterized in that** the rib (1a) and the third hole (1a1) located in the middle part are located below an attachment hole among the first and second attachment holes (1b) of the plate.

10. The implant according to any one of the preceding claims, **characterized in that** the first and second attachment holes (1b) of the plate are threaded.

11. The implant according to any one of the preceding claims, **characterized in that** additional attachment holes are provided in the thickness of the plate (1) between or outside the first and second attachment holes (1b).
